# EUROPEAN PATENT SPECIFICATION

(11) **EP 4 178 634 B1**
(45) Date of publication and mention of the grant of the patent: **17.07.2024**
(21) Application number: 21752160.8
(22) Date of filing: 08.07.2021
(51) Int. Cl.: A61L 27/18, A61L 27/34, A61L 27/38, A61L 27/56

(54) **INTEGRATED CORE-SHELL BIOACTIVE STRUCTURE FOR THE REGENERATION OF BONE AND OSTEOCHONDRAL TISSUES**
INTEGRIERTE BIOAKTIVE KERN-SCHALE-STRUKTUR ZUR REGENERATION VON KNOCHEN- UND OSTEOCHONDRALEN GEWEBEN
STRUCTURE BIOACTIVE À NOYAU-ENVELOPPE INTÉGRÉE POUR LA RÉGÉNÉRATION DE TISSUS OSSEUX ET OSTÉOCARTILAGINEUX

(30) Priority: 08.07.2020 IT 202000016579
(43) Date of publication of application: 17.05.2023
(73) Proprietor: Universita' degli studi di Brescia, 25121 Brescia (IT); Le Rondini - Citta' Di Lumezzane Onlus, 25065 Lumezzane (IT); Fondazione della Comunita' Bresciana Onlus, 25122 Brescia (IT)
(72) Inventor: SARTORE, Luciana, 36035 Marano Vicentino (IT); RUSSO, Domenico, 25133 Brescia (IT); PANDINI, Stefano, 25133 Brescia (IT); NICOLAI, Piero, 46049 Volta Mantovana (IT); FERRARI, Marco, 35126 Padova (IT); GILBERT, Ralph, Toronto, Ontario M4X 1L1 (CA); IRISH, Jonathan, Toronto, Ontario M4S 151 (CA)
(74) Representative: Palladino, Saverio Massimo
(86) International application number: PCT/IB2021/056113
(87) International publication number: WO 2022/009126

(56) References cited:
- MEHR NIMA GHAVIDEL ET AL: "Pore size and LbL chitosan coating influence mesenchymal stem cell in vitro fibrosis and biomineralization in 3D porous poly(epsilon-caprolactone) scaffolds : BIOMINERALIZATION IN CHITOSAN-COATED 3D PCL", JOURNAL OF BIOMEDICAL MATERIALS RESEARCH PART A, vol. 103, no. 7, 29 December 2014 (2014-12-29), US, pages 2449 - 2459, XP055785293, ISSN: 1549-3296, DOI: 10.1002/jbm.a.35381
- RE FEDERICA ET AL: "3D gelatin-chitosan hybrid hydrogels combined with human platelet lysate highly support human mesenchymal stem cell prolifeartion and osteogenic differentiation", 1 January 2019 (2019-01-01), pages 1 - 16, XP055784431, Retrieved from the Internet <URL:https://journals.sagepub.com/doi/pdf/10.1177/2041731419845852> [retrieved on 20210311]

## Description

### FIELD OF THE INVENTION

The present invention refers to an integrated core-shell bioactive structure which is used in the field of tissue engineering to promote tissue regeneration, in particular of bone and osteochondral tissues; the invention also relates to the method for producing the structure.

### STATE OF THE ART

In many situations it is necessary to replace or integrate portions of bone or cartilage tissue of the human or animal body, or facilitate the regeneration thereof.

Bone loss can result from multiple causes including trauma, blast injuries, diseases such as osteomyelitis, osteonecrosis or osteosarcoma, or surgical excisions. Such conditions often result in cavitation or complete loss of bone tissue, and bone repair or regeneration in these cases become difficult, very long in time, or sometimes impossible. Injury or loss of cartilage tissue can instead be a consequence of trauma or physiological wear, especially in athletes or people whose joint cartilages are in any case subjected to intense and prolonged stresses.

The treatment of bone and cartilage defects is still an unsolved problem. Bone tissue shows limited regeneration and repair properties due to the extracellular matrix characteristics and the lack of blood and lymphatic flows essential for tissue regeneration processes. Consequently, bone lesions other than simple fractures are significantly difficult to treat.

In the case of hyaline cartilage, the problem is enhanced because this tissue, in addition to lacking blood vessels and the lymphatic system, is also devoid of nerves. Hyaline cartilage has limited reparative capacity, and the generated fibrocartilage repair tissue lacks the structure and biomechanical properties typical of healthy cartilage and degrades over time. Therefore, injuries or degenerations of the articular cartilage generally do not heal, or heal only partially.

Many techniques have been used for the purpose of attempting to repair these defects.

In the case of bone tissue, the most common ones include bone replacement with autologous vascularized bone grafts (autotransplant), massive allograft (generally from a cadaver), or the use of resorbable or non-resorbable "artificial bone".

Another method to promote bone regeneration is through the introduction of osteoinductive bioactive factors (bone morphogenetic proteins, plasma rich in platelets, synthetic peptides, *etc*.)*,* that can be introduced into the area of bone loss through various techniques. Mechanical methods are also used to promote bone regeneration, such as distraction osteogenesis and guided or protected bone regeneration.

In the case of injuries and degenerations of the articular cartilage, the repair techniques currently most used clinically are not aimed at replacing the cartilage but rather at arthroscopic washing and debridement (*i*.*e*., removal of tissue fragments generated by the trauma that led to the injury), and repair stimulation.

Arthroscopic washing involves irrigating the joint with solutions that eliminate degenerative debris arising from the cartilage and can provide temporary relief from pain, but have little or no opportunity of definitive repair and healing.

Repair stimulation is performed by perforation, arthroplasty abrasion or microfracture. The penetration of the subchondral bone, in fact, induces bleeding and formation of fibrin clots which favour the initial repair of the cartilage; however, the formed tissue is fibrous and non-durable in nature, it wears over time, degenerates, and loses resilience and stiffness. In fact, regenerated cartilage always shows characteristics of fibro-hypertrophic cartilage tissue.

Therefore, although different methods and systems are currently known to deal with bone and joint problems, the treatment of injuries with severe bone loss, the treatment of articular cartilage degeneration and, in general, the treatment of fractures that do not heal spontaneously remain a difficult clinical problem, and the need still exists for the least invasive possible effective methods of promoting bone growth.

Tissue engineering represents a possible solution for the repair and regeneration of these tissues. This branch of biomedical engineering deals with identifying systems that can be produced in laboratory and that are able, once inserted into the site of a lesion, to stimulate the regrowth of damaged tissue. Parts that are implanted in the human or animal body in order to, at least temporarily, compensate for a compromised function and stimulate tissue regeneration are called in the medical field with the English term "scaffold", which will also be used in the present description.

Various systems based on tissue engineering have been described in the medical and patent literature. These systems are often based on the production of more or less ordered skeins of polymer filaments produced by electrospinning, optionally in a core-shell configuration; the English definition core-shell, generally means a structure consisting of at least two different materials, wherein one of the two materials forms an internal skeleton whose surface is coated by the second material. In the case of electrospinning, the core-shell filaments consist of a central part of a first polymer coated by a sheath of a second polymer; the two polymers are co-produced during the same electrospinning phase and have to be chemically compatible to avoid demixing of the materials and delamination of the sheath from the core. The filaments generally have diameters that can vary between a few tens of nanometers and about one micrometer; in the skeins produced by electrospinning, the filaments are intertwined but spaced apart, so as to leave empty voids that can be colonized by cells of the tissue whose regrowth is to be promoted.

This approach is followed, for example, in the article "Potential core-shell designed scaffolds with a gelatin-based shell in achieving controllable release rates of proteins for tissue engineering approaches", F. Ghasemkhah et al., J Biomed Mater Res Part A 2019:107A:1393-1405. This article describes the production of bicomponent fibers, consisting of a bovine serum albumin core and a gelatin shell; the fiber structure is consolidated by cross-linking with glutaraldehyde; the application of the scaffolds in this article is generic, and not directed to regrowth of a specific tissue.

The article "Hybrid core-shell scaffolds for bone tissue engineering", Muna M. Kareem et al., Biomed. Mater. 14 (2019) 025008 describes the production of scaffolds for bone regrowth consisting of fibers made with a polycaprolactone core coated with a polylactic acid shell loaded with hydroxyapatite nanoparticles.

The article "Pore size and LbL chitosan coating influence mesenchymal stem cell in vitro fibrosis and biomineralization in 3D porous poly(epsilon-caprolactone) scaffolds", N. G. Mehr et al., J. Biomed. Mater. Res. A vol. 103 no. 7 (2015), pages 2449-59, describes a 3D porous scaffold made of polycaprolactone whose surface is coated with chitosan by layer-by-layer (LbL) polyelectrolysis.

Finally, patent application CN 105688274 A describes the electrospinning production of skeins of polycaprolactone fibers coated with gelatin, on whose exposed surface hydroxyapatite nanocrystals are present.

The systems of these documents, made of fibers of very thin diameter, generally have the problem of not exhibiting high mechanical characteristics; they are able to stimulate cell regrowth, but do not compensate for the bone tissue mechanical function.

Another limitation of these systems is that they are generally produced with pairs of chemically compatible materials to ensure adhesion of the shell to the core; this greatly limits the freedom of choice of the two materials, and therefore the possibility of modulating the set of characteristics, in particular the mechanical ones, of the scaffold produced with these fibers.

The object of the present invention is to provide an integrated core-shell bioactive structure for the regeneration of bone and osteochondral tissues, as well as to provide a process for the production thereof.

### SUMMARY OF THE INVENTION

These objects are achieved with the present invention, which in its first aspect relates to an integrated core-shell bioactive structure for the regeneration of bone and osteochondral tissues, consisting of:
- a porous core made of a biocompatible and biodegradable thermoplastic polymer selected from polylactic acid, polyglycolic acid, polycaprolactone and mixtures thereof, in an amount between 1 and 99% by weight of the structure; and
- a core coating, in an amount between 1 and 99% by weight of the structure, comprising a hydrogel formed by reaction of a polypeptide either derived from natural tissues or synthetic, a water-soluble polymer functionalized so as to be capable of reacting with the polypeptide, and an optionally aminated polysaccharide, with a weight average molecular weight greater than 3 kDa.

The core coating of the structure according to the first aspect of the invention may further contain one or more additional components selected from:
- mesenchymal stromal cells deriving from bone marrow, adipose tissue or umbilical cord;
- growth factors;
- antibiotics, drugs or medicines useful in the treatment of bone lesions and/or in the regeneration of tissues;
- additives for modifying the stiffness or degradability of the structure, such as complexes of calcium, calcium phosphate, calcium carbonate, decellularized and pulverized bone material and hydroxyapatite.

In its second aspect, the invention relates to a process for the production of the structure described above, which includes the following steps:
a) providing a porous body made of polylactic acid, polyglycolic acid, polycaprolactone or a mixture thereof, which has open and interconnected porosities throughout the body itself and a porosity of between 30 and 95%
b) preparing an aqueous solution comprising a polypeptide either derived from natural tissues or synthetic, a water-soluble polymer functionalized so as to be capable of reacting with the polypeptide, and an optionally aminated polysaccharide, with a weight average molecular weight greater than 3 kDa, and allowing the system to react under stirring at a temperature between 20 and 70 °C for a time between 10 minutes and 1 hour;
c) immersing the core prepared in step a) in the solution prepared in step b) and then degassing the solution by subjecting it to at least one evacuation cycle at a pressure P ≤ 0.1 mbar and exposure to an inert gas atmosphere, then maintaining the solution in said inert atmosphere at a temperature between 20 and 70 °C for a time between 30 minutes and 2 hours;
d) extracting the core from the still fluid solution and allowing the layer of solution adherent to the core surface to gel, or waiting for the solution to gel and extracting a hydrogel incorporating the core from the container of the initial solution;
e) freezing the body consisting of the core coated by wet hydrogel at a temperature below -4 °C and freeze-drying obtaining the hydrogel drying;
f) completing the hydrogel cross-linking reaction in an oven under vacuum at a temperature between 30 and 70 °C for a time between 2 and 6 hours.

### BRIEF DESCRIPTION OF THE FIGURES

The invention will be described with reference to the Figures, in which:
- Fig. 1 reproduces two photographs, at different magnifications, of a porous body that can be used as core in a structure of the invention;
- Fig. 2 reproduces a photograph of a section of a sample of the structure of the invention;
- Fig. 3 reproduces a photograph obtained with a scanning electronic microscope on the surface of the sample shown in Figure 2;
- Fig. 4 shows graphs relating to mechanical tests carried out on a sample of the structure of the invention;
- Fig. 5 shows in a graph the trend of the amount of live and dead cells in a sample of the structure of the invention, as a function of the distance from the external surface of the structure;
- Fig. 6 shows in a graph the results of the trend over time of the relative density of surgical sites in which samples of the structure of the invention and comparison structures have been implanted.

### DETAILED DESCRIPTION OF THE INVENTION

In the description and claims, unless otherwise noted, component amounts and solution concentrations are reported in percentages by weight.

The expression "long chain", referred to the polysaccharide used as the third component of the coating in the structures of the invention, means a polymeric carbohydrate having a weight average molecular weight of at least 3 kDa.

In its first aspect, the invention relates to an integrated core-shell bioactive structure that is used in the field of tissue engineering, capable of promoting tissue regeneration, in particular, of bone and osteochondral tissues, and having sufficient mechanical consistency to be able to compensate for the function of the bone tissue until complete regrowth of the same; the invention also relates to the method for producing the structure.

The structure has a core-shell configuration, wherein the core consists of a porous body made of thermoplastic polymeric material that provides the structure with shape and mechanical strength, and the shell consists of a hydrogel that fully covers the core, both the external surface and the surface of the internal pores thereof. The structure of the invention differs from similar known structures because the latter are made with chemically compatible polymers, while a shell made with a hydrogel would not be compatible with the core polymer and would be washed away by water (or aqueous solutions) during the production treatments of the final structure; in the case of the present invention, the shell has a modified chemical composition which allows its stable adhesion to the core.

The porous core can be produced according to two methods.

In a first method, the core is produced by dispersion of particles in the polymeric melt which will form the final core by means of a Brabender type batch mixer (or by extruder). The dispersed particles are made of a cross-linked polymer, infusible at the core melting temperature and characterized by very high water absorption (superabsorbent SAP) but insoluble in water, or particles of a second material having a melting temperature higher than that of the core polymer and water-soluble, or a mixture of these two types of particles. When the core polymer is melted, the mixture containing the particles of said second material is mixed thanks to the action of the rotors to uniformly disperse the solid particles in the melt. The composite material obtained is moulded in the desired shape and size, and finally the particles of the second material are washed away by immersion in water, which interacts directly with the dispersed particles. In the case of soluble particles, this interaction simply consists in their dissolution. In the case of SAP particles, the mechanism of interaction with water is different: the particles swell when in contact with water, and those exposed to the surface are pushed out of the structure by the pressure they exert against the walls of the core; water penetrates inside the pores thus created and comes into contact with other SAP particles, which in turn swell and, by pressure against the rigid parts of the structure, are pushed into the adjacent open pores; this mechanism is repeated until the SAP particles are completely ejected from the core. This procedure leads to the formation of the porous structure of the core.

As mentioned, the core material is selected from polylactic acid, polyglycolic acid, polycaprolactone or a mixture thereof; the three polymers are generally referred to in the field with the abbreviations PLA, PGA and PCL, respectively, which will also be used in the remainder of this description.

The choice of the second material, in the form of particles, depends on the amount that can be tolerated by the polymer melt and on the degree of porosity to be obtained. In the case of superabsorbent polymer, this is preferably cross-linked sodium polyacrylate; particles of this polymer have a very high swelling in water before leaking out of the matrix, and a porosity of 60% is obtained with a percentage of 30% by weight. In the case of particles which are simply soluble in water, these will generally be made of inorganic salts; by operating according to this second possibility, lower porosity values, indicatively between 5 and 50%, are obtained.

To obtain porosities suitable for the purposes of the present invention, the particles of the second material should have a particle of size between 5 and 200 µm, and should be used in a weight ratio between particles and core polymer of between 10:90 and 50:50.

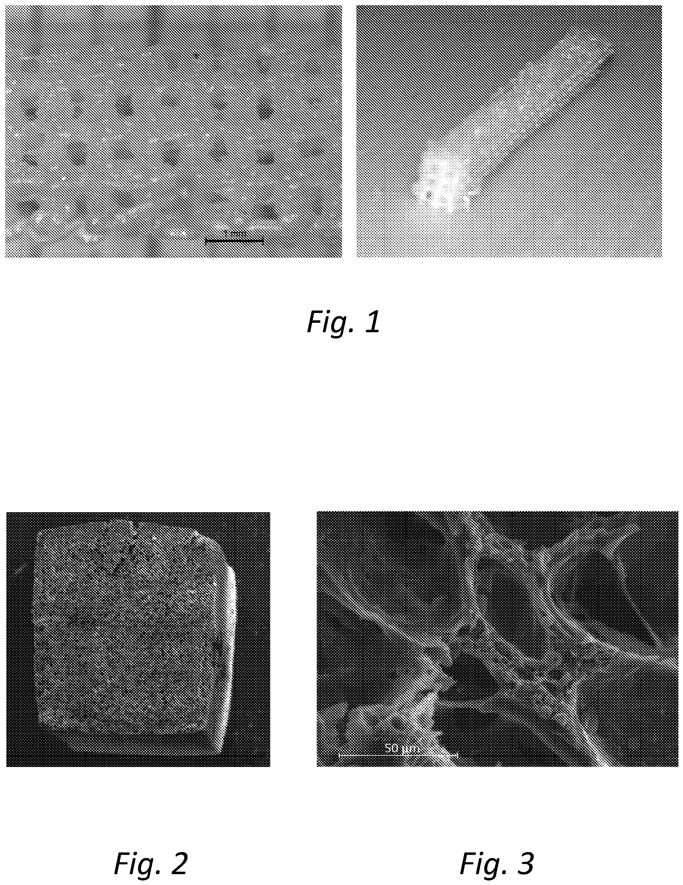

In the second method, the core can be produced using 3D printing techniques. In this technique (also known as additive manufacturing), as known, the material used to produce a three-dimensional object is melted in a tank and deposited in successive layers through a nozzle; the deposition area of each individual layer is controlled by a computer which moves the nozzle in an x-y plane according to a CAD drawing, and at each layer raises the nozzle along the z axis perpendicular to the x-y plane. An example of a core structure obtained according to this second method is shown in the two photographs, at different magnifications, reproduced in Fig. 1.

The shell material comprises a hydrogel, formed by reaction (cross-linking) of three polymeric components, namely a polypeptide, a water-soluble polymer and a long-chain polysaccharide.

The first component of the shell, the polypeptide, can be derived from natural tissues or be synthetic; it is also possible to use a mixture of natural and synthetic polypeptides. Natural polypeptides can be of animal or plant origin. The polypeptide is preferably a gelatin produced through the hydrolysis of collagen extracted from skin, bones and connective tissues of animal waste. This natural component is widely available commercially. The polypeptide is present in the shell material in an amount between 48.6% and 80.2% by weight, and preferably between 60% and 70%, calculated on the sum of the polypeptide, water-soluble polymer and polysaccharide components.

The second component of the shell is a water-soluble polymer such as polyethylene glycol (PEG), polyvinylpyrrolidone (PVP) and polyvinyl alcohol (PVA); the water-soluble polymer is functionalized with groups capable of reacting with reactive sites of the polypeptide. The preferred water-soluble functionalized polymer for the purposes of the invention is polyethylene glycol diglycidyl ether, compound of the formula: 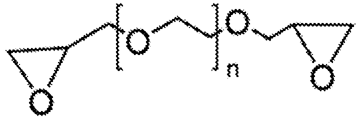 wherein n is an integer lower than 30 and preferably between 5 and 9. This component is present in the shell in an amount between 11.4% and 18.8% by weight, and preferably between 14% and 17%, calculated on the sum of the polypeptide, water-soluble polymer and polysaccharide components.

The third component of the shell is defined in the claims and is a long-chain polysaccharide preferably selected from optionally aminated dextran and chitosan. Dextran is a branched polymer produced by fermenting glucose by action of lactobacilli or other fermenting bacteria. Chitosan is a linear polysaccharide obtained by deacetylation of chitin (generally extracted from crustacean exoskeleton) in a basic aqueous solution. Both dextran and chitosan are commonly used in the industry, for example in the cosmetic, pharmaceutical or food additive sectors, and are commercially available. The preparation of the polysaccharide in aminated form occurs by activating the polysaccharide with 4-nitrophenylchloroformate and subsequent reaction with ethylenediamine. This component is present in the shell material in an amount between 1% and 40% by weight, and preferably between 10% and 25%, calculated on the sum of the polypeptide, water-soluble polymer and polysaccharide components.

A preferred composition for producing the shell of the invention comprises 66% of polypeptide, 16% of functionalized water-soluble polymer and 18% of polysaccharide, by weight.

As discussed below with reference to the process of the invention, these three components cross-link each other forming a hydrogel that can then be dried forming the core coating.

Additional components useful for promoting the regrowth of bone or osteochondral tissues, or for modulating the stiffness or biodegradability of the porous body itself, can be introduced into the shell material.

A first possible additive are mesenchymal stromal cells deriving from bone marrow, adipose tissue or umbilical cord, which have the function of favouring and accelerating the osteo-inductive capacity of the scaffold. These cells can be added in amounts between 1000 and 10000 cells/mm³ of the porous body.

A second useful additive is made of growth factors, that is proteins specialized in stimulating cell proliferation and differentiation. Particularly useful for the purposes of the invention are the human platelet lysate (HPL), the bone morphogenetic protein (BMP), which stimulates the differentiation of osteoblasts, and the vascular endothelial growth factor (VEGF) which stimulates the growth of vessels and therefore promotes the vascularization of the regrowing bone tissue.

A third possible additive are drugs or medications useful in the treatment of bone lesions and/or tissue regeneration, such as antibiotics and platelet growth factors. The amount of these additives depends on both the recipient (age, body weight, *etc.*) and the type of medicine, and it is a parameter that can be easily determined by medical personnel.

Finally, additives capable of modifying the stiffness or biodegradability of the porous body can be added to the porous body. These additives can be complexes of calcium, calcium phosphate, or nanometric hydroxyapatite powders.

In the core-shell structure of the invention, the weight ratio between core can vary within wide limits: each of the two components can constitute from 1 to 99% by weight of the structure. Structures with a high percentage by weight of core are more suitable for bone tissue regeneration; conversely, structures in which the shell component is predominant are more suitable for use in joint tissue or soft tissues regeneration.

The core-shell structure of the invention exhibits a set of optimal characteristics for a scaffold for bone or osteochondral tissue regrowth:
- pore sizes in the range between a few tens of µm and about 500 µm. In various studies it was determined that these sizes, in particular between about 100 and 500 µm, are optimal for bone regrowth because they represent an ideal compromise between the need to have a size sufficient for vascularization of the regrowing tissue but not such as to make colonization of the central portion of the pores difficult. The pore sizes can be modulated by controlling the size and amount of material particles added and then selectively eliminated during core production. Furthermore, in devices in which the core is completely incorporated in the shell, the porosity can be modulated with the dilution of the hydrogel solution, the type and molecular weight of the polysaccharide and the cross-linking density;
- a porosity structure such that the pores are homogeneously distributed throughout the material, in communication and well interconnected with one another. This structure is highlighted in the photographs at different magnifications, obtained with an optical or electronic microscope, and reproduced in Figures 2 and 3, respectively;
- mechanical characteristics compatible with the intended application and modulable depending on the composition;
- thanks to the presence of the two different materials, the core-shell structure allows rapid colonization by human mesenchymal cells, osteogenic differentiation with a relatively rapid metabolization of the shell material, and mechanical support to the regrowing tissue for longer times, since the core material has a slower resorption kinetics than that of the shell;
- following dedicated tests, the scaffolds of the invention showed homogeneous cell colonization on the surface and inside the scaffold, excellent characteristics of cell adhesion to the pore walls, stimulation of cellular colonization of the pores themselves and properties as inducers of osteogenesis and osteochondrogenesis;
- finally, the scaffolds of the invention showed complete metabolization and resorption in times between 4 and 24 months, depending on their composition.

In its second aspect, the invention relates to a process for producing the core-shell structure described above, comprising the steps a) to f) mentioned above.

Step a) is carried out with one of the two methods described above, *i.e.* removal by selective leaching of solid particles incorporated in a matrix of PLA, PGA, PCL or mixtures thereof; or, alternatively, by forming with 3D printing techniques starting from the same polymers.

Step b) is carried out with the following series of operations:
b.1) dissolving the polypeptide in distilled water, at a temperature between 20 and 70 °C;
b.2) adding the functionalized water-soluble polymer to the solution obtained in step a) and allowing the system to react at a temperature between 20 and 70 °C for a time between 5 and 30 minutes;
b.3) adding the long-chain polysaccharide, selected from optionally aminated dextran and chitosan or a mixture thereof, to the solution obtained in step b.2), and allowing the system to react at a temperature between 20 and 70 °C for a time between 5 minutes and 1 hour.

Step c) of the method of the invention consists in immersing the core obtained in step a) in the solution prepared in step b), and then degassing the solution by subjecting it to at least one evacuation cycle at a pressure P ≤ 0.1 mbar and exposure to an inert gas atmosphere, then maintaining the solution in said inert atmosphere at a temperature between 20 and 70 °C for a time between 30 minutes and 2 hours. In this period of time, the hydrogel cross-linking and grafting of the hydrogel onto the porous core take place. In fact, the functionalized PEG, in excess with respect to the amino groups of the polypeptide, is reactive towards a multiplicity of functional groups (primary and secondary amines, carboxylic acids, hydroxyls, etc.) and gives rise to grafting and exchange reactions with the thermoplastic matrix.

The conditions and methods of execution of this step determine the obtainment of different final core-shell structures. When operating at relatively high temperatures in the aforementioned range, in particular above 40 °C, the solution always remains fluid, albeit of high viscosity, for the entire time of immersion of the porous core in it; in this case, when the core is extracted from the solution, this partly flows from the core pores, but a layer of solution remains adherent to the surface (external and internal) thereof, due to grafting and exchange reactions with the surface of the core (which determine the grafting); this layer of solution is then gelified afterwards. Conversely, when operating at low temperatures in the indicated range, the solution gelifies completely and a body consisting of a hydrogel matrix, containing the core inside it, is extracted from the container initially containing it; when operating according to this second possibility, it is preferable that the container initially containing the solution of point b) has shape and size similar to those of the final core-shell structure desired.

During the immersion period of the core in the solution, this is subjected to at least one cycle (preferably 2 or 3 cycles) of evacuation at reduced pressure and subsequent exposure to an inert atmosphere. Evacuation, carried out at values of P ≤ 0.1 mbar, is possible thanks to the high viscosity of the solution, which prevents it from evaporating or being sucked by the vacuum system. The evacuation phase (or phases) causes the extraction of air from the pores of the core; the subsequent phase (or phases) of exposure to an inert atmosphere reestablish the pressure on the solution thus pushing it inside the evacuated pores. This procedure ensures the complete filling of the pores with the solution which otherwise, due to its high viscosity, would not be able to penetrate the same pores.

In step d) the core coated with said solution, or a hydrogel incorporating it, is extracted from the solution container. In the first case, the system thus obtained is allowed to rest for a time between 30 minutes and 6 hours at a temperature below 30 °C, obtaining a wet gel that covers the external and internal walls of the core; in the second case, the body extracted from the container already consists of a hydrogel incorporating the core, and is ready for the next operation.

In step e) of the process, the assembly consisting of the core coated with wet gel is subjected to a treatment for freeze-drying the hydrogel, which consists in freezing the system at a temperature below -4 °C and then sublimating the water present in the system at a pressure lower than 0.1 mbar, obtaining a dry gel which forms the shell of the structure of the invention.

Finally, in the last step of the process, f), the porous core coated with the dry gel obtained in the previous step is treated in oven under vacuum (P ≤ 0.1 mbar) at a temperature between 30 and 70 °C for a period of between 2 and 6 hours. In this step, the cross-linking reactions among the components of the dry gel, and between these and the thermoplastic polymer of the core, are completed, obtaining a core-shell structure wherein the two parts are perfectly integrated and adherent.

As previously said, the methods of execution of step c) determine the type of core-shell structure obtained at the end of the process. Structures having a high percentage by weight of core material, suitable for bone tissue regrowth, can be obtained by extracting the core-shell structure from the warm hydrogel solution before gelling; conversely, structures having a high percentage by weight of shell material, more suitable for joint tissue regrowth, can be obtained by allowing the solution to gel on the porous core before its extraction, even until complete incorporation of the core is obtained.

The core-shell structure thus obtained is preferably sterilized, and can be stored in gas-tight containers or bags for periods of months or even a few years.

Before use, the operator can extract the core-shell structure from the container, optionally shape it to adapt it to the implant site, and hydrate it with water or physiological solution before implantation in the body. In this phase, it is possible to add some additional components mentioned above (dissolved in water or physiological solution, or at a later stage), *i.e.* mesenchymal stromal cells from bone marrow, growth factors, drugs or medicines useful in the treatment of bone lesions and/or in tissue regeneration, and additives that modify the stiffness or degradability of the structure.

The invention will be further illustrated by the following examples.

### Methods, instruments and materials

An electromechanical dynamometer (Instron Model 3366) was used for the mechanical characterizations (compression and traction tests).

The molecular weight of the polymers was determined by gel permeation chromatography (GPC) (Erma Inc. chromatograph) and Shodex KF columns. The calibration curve was obtained with 16 narrow distribution polystyrene standards (Polymer Laboratories) with molar mass between 3,18 × 10⁶ and 162 g/mol.

An Eclipse 90i microscope (Nikon Instruments Europe BV) and a Leica CM 1860 ultraviolet (UV) cryostat were used for the biological characterizations.

The following materials were used in the tests:
- Polylactic acid: weight average molecular weight (M_{w}) 199,590 Da, Nature Works (Blair, NE);
- Polycaprolactone: number average molecular weight (Mn) 10,000 Da, Sigma-Aldrich Co, Milan;
- Cross-linked sodium polyacrylate (SAP, Produkt T 5066 F), particle size lower than 63 µm and density of 0.7 g/cm³, Evonik Industries AG (Essen, Germany);
- Type A Gelatin, pharmaceutical grade, 280 bloom, viscosity 4.30 mPs, Italgelatine, (Cuneo, Italy);
- Polyethylene glycol diglycidyl ether, molecular weight 526 Da, Sigma-Aldrich Co, Milan, Italy;
- Dextran Mw 70.000 Da, Sigma-Aldrich Co, Milan, Italy;
- 4-Nitrophenyl chloroformate Sigma-Aldrich Co, Milan, Italy;
- Ethylenediamine Sigma-Aldrich Co, Milan, Italia;
- Growth medium with 10% fetal bovine serum (GM FBS): Dulbecco's modified Eagle's medium (DMEM), l-glutamine, penicillin-streptomycin, sodium pyruvate (Sigma-Aldrich Co, USA), amphotericin and non-essential amino acids (Gibco, ThermoFisher Scientific, USA), fetal bovine serum (Sigma-Aldrich Co, USA);
- Growth medium with 5% platelet lysate (GM HPL): Dulbecco's modified Eagle's medium (DMEM), L-glutamine, penicillin-streptomycin, sodium pyruvate (Sigma-Aldrich Co, USA), amphotericin and non-essential amino acids (Gibco, ThermoFisher Scientific, USA);
- Osteogenic medium with 10% fetal bovine serum (OM FBS): Dulbecco's modified Eagle's medium (DMEM), L-glutamine, penicillin-streptomycin, sodium pyruvate (Sigma-Aldrich Co, USA), amphotericin and non-essential amino acids (Gibco, ThermoFisher Scientific, USA), fetal bovine serum, dexamethasone, L-ascorbic, NaH₂PO₄ (Sigma-Aldrich Co, USA);
- Osteogenic medium with platelet lysate (OM HPL): Dulbecco's modified Eagle's medium (DMEM), L-glutamine, penicillin-streptomycin, sodium pyruvate (Sigma-Aldrich Co, USA), amphotericin and non-essential amino acids (Gibco, ThermoFisher Scientific, USA), dexamethasone, L-ascorbic, NaH₂PO₄ (Sigma-Aldrich Co, USA);
- Phosphate buffered saline (PBS) (Sigma-Aldrich Co, USA);
- 4% Paraformaldehyde (PFA) (Sigma-Aldrich Co, USA);
- 4',6-Diamidine-2-phenylindole (DAPI) (Vector Laboratories, USA);
- Mesenchymal stromal cells from bone marrow (BM-hMSCs) and adipose tissue (AT-hMSCs) (PromoCell, Germany).

### EXAMPLE 1

This example refers to the preparation of a first PLA porous core.

40 grams of PLA granules, previously dried in an oven at 70 °C, were mixed with 17.1 grams of cross-linked sodium polyacrylate particles having a particle size lower than 63 µm. The mixture was treated at 180 °C in a batch mixer (Brabender, Plastograph, Duisberg, Germany) for 6 min with a screw speed of 50 rpm; a melted sample is obtained inside which the sodium polyacrylate particles are dispersed. The sample was collected and then moulded with a compression press (Collin P200 E) at a temperature of 180 °C and a pressure of 30 bar, obtaining sheets with a thickness of 3 mm from which bodies having parallelepiped geometry, with dimensions equal to 3 × 3 mm and variable length from 5 to 30 mm, are cut.

These bodies were then immersed in water for 7 days with the aim of promoting complete swelling and leaching of the sodium polyacrylate particles. The material thus obtained exhibited a porosity P = 59.9%, measured by a method known in the literature as the "liquid substitution method". The specimens were washed, dried and vacuum sealed in plastic bags. All the specimens were sterilized by gamma radiation, Co 60 dose 25-33 kGy (UNI EN ISO 11137 - Sterilization of Health Care Products). These specimens form Sample 1.

### EXAMPLE 2

This example refers to the preparation of a PLA-PCL porous core.

The procedure of Example 1 was repeated, with the only difference that a mixture consisting of 45.6 g of PLA and 11.4 g of PCL was used as the material for producing the porous core. The material thus obtained exhibited a measured porosity equal to P = 61.1%. The specimens thus obtained form Sample2.

### EXAMPLE 3

This example refers to the preparation of a second PLA porous core.

In this case the porous core was produced with 3D printing, obtaining a sample (shown in the two photographs of Fig. 1) of dimensions 3 × 3 × 30 mm with pores of approximately square section with side of about 300 µm. The PLA was printed at 190 °C. This specimen forms Sample 3.

### EXAMPLE 4

This example refers to the preparation of a core-shell structure of the invention.

A hydrogel solution was prepared according to the following procedure. 6 g of gelatin were dissolved in 65 ml of water at 40 °C under magnetic stirring; 1.4 g of polyethylene glycol diglycidyl ether and then 0.8 g of chitosan were added dropwise, keeping the reaction mixture at 45 °C and under stirring for a further 15 min.

The specimens of Sample 2 were immersed in the solution thus obtained. The solution with the immersed specimens was subjected to three successive vacuum and filling cycles with an inert nitrogen atmosphere, always maintaining the temperature at 45 °C.

The bars were then extracted from the solution, frozen and freeze-dried. The specimens were then treated in an oven at 50 °C for 3 hours and then vacuum sealed in plastic bags and sterilized by gamma radiation, Co 60 dose 25-33 kGy (UNI EN ISO 11137 - Sterilization of Health Care Products). These bars form Sample 4.

### EXAMPLE 5

One of the specimens of Sample 2 and one of the specimens of Sample 4 were fully characterized in order to determine their chemical composition and technical characteristics. By means of FTIR spectroscopy, it was possible to ascertain the presence of all the components and, in particular, of the hydrogel. For comparison purposes, a specimen made with hydrogel only was also produced, according to the production methods of this component described above (this sample is referred to below as Hy). By coupling this technique with TGA and DSC, the composition of the materials was determined. Table 1 shows the composition of the tested specimens and the average, minimum and maximum size of the pore diameters evaluated with microscopic techniques; pore size assessment was performed on dry materials.

**Table 1**

| Material | Composition (%) | | | D_{average} pores (µm) | Dmin÷Dmax (µm) |
|---|---|---|---|---|---|
| | Core | | Shell | | |
| | PLA | PCL | Hy | | |
| Hy | - | - | 100 | 140 ± 50 | 70÷260 |
| Sample 2 | 80 | 20 | - | 76 ± 27 | 40÷170 |
| Sample 4 | 78 | 19.5 | 2.5 | 100 ± 29 | 70÷140 |

Figures 2 and 3 show, respectively, an optical microscope photograph and an electronic microscope photograph obtained on a section of Sample 4.

The material exhibits very high porosity, pores of heterogeneous size but homogeneously distributed and well interconnected. Small, rounded domains of PCL (biphasic core) and a smooth hydrogel coating (shell) covering the inner pore wall are also visible.

### EXAMPLE 6

One of the specimens of Sample 2 and one of the specimens of Sample 4, both of dimensions 3 × 3 × 30 mm, were characterized by traction, compression and bending mechanical tests.

Table 2 reports the mechanical characteristics of the specimens, which show that the core-shell scaffold can be stressed in various ways while maintaining good strength and has a high stiffness comparable to that of spongy bone tissue. Furthermore, the final stiffness of the material can be modulated by varying the compositional ratios.

**Table 2**

| Sample | Traction | | Compression | | Bending | |
|---|---|---|---|---|---|---|
| | Elastic Modulus [MPa] | Maximum stress [MPa] | Elastic Modulus [MPa] | Maximum stress [MPa] | Elastic Modulus [MPa] | Maximum stress [MPa] |
| Hy | 1.90 ± 0.3 | 0.96 ± 0.21 | 0.30 ± 0.01 | 0.04 ± 0.01 | / | / |
| Sample 2 | 97 ± 18 | 1.7 ± 0.6 | 43.2 ± 3.1 | 5.2 ± 0.9 | 92 ± 13 | 3.3 ± 0.5 |
| Sample 4 | 103 ± 17 | 0.9 ± 0.2 | 26.0 ± 6.2 | 4.6 ± 1.4 | 205 ± 17 | 2.9 ± 0.2 |

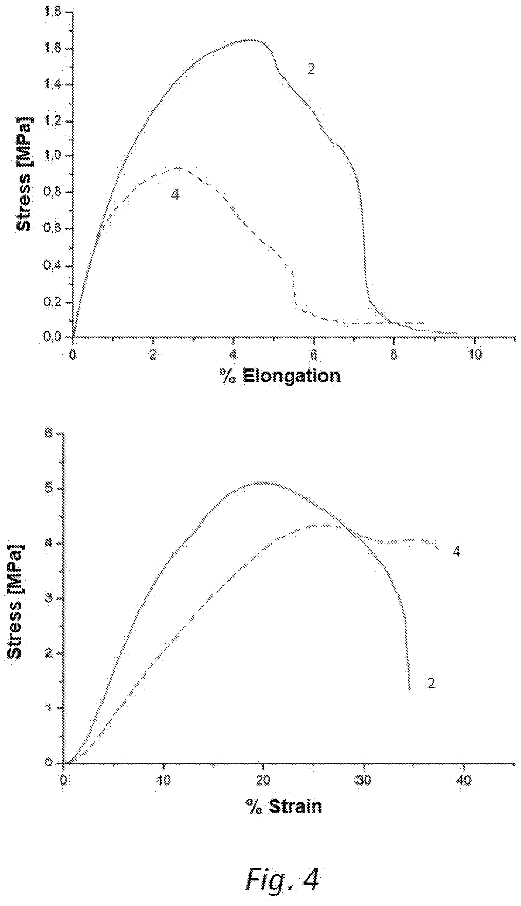

The upper part of Figure 4 shows the curves obtained in the traction test on Samples 2 and 4, and the lower part of the figure shows the curves obtained in the compression tests on the same two Samples; in both figures, the solid curve refers to Sample 2 while the dashed curve refers to Sample 4 (as also indicated by the numbers in the figures).

### EXAMPLE 7

In this example, the proliferation of human stem cells (hMSCs) on a scaffold of the invention is evaluated - *In vitro* test.

A specimen of Sample 4 with dimensions of 3 × 3 × 5 mm, prepared and sterilized as described in Example 4, was used as a scaffold. The specimen was hydrated for 24 h in a solution of "Dulbecco's Modified Eagle Medium" (DMEM, Sigma Aldrich^{®}) with 5% human platelet lysate (HPL), and cell culture incubation conditions (37 °C, 5% CO₂) and subsequently seeded with human mesenchymal stem cells (hMSC) at step 2 (P2) by immersion in culture medium (DMEM + 5% HPL) with a concentration of 2,000 cells/mm³, for a duration of 24 hours and incubation conditions from cell culture (37 °C, 5% CO₂). The time of immersion of the scaffold in the culture medium with hMSC was considered to be time 0. The culture medium was replaced every 24 hours under sterile conditions. On day 4 (96 hours after seeding) the scaffold was subjected to an *in vitro* cell viability study, extracting it from the culture medium and subjecting it to viability assay by staining with calcein and propidium iodide (ThermoFisher^{®}) and NucBlue^{®} (Invitrogen^{®}) and subsequent scanning by 3-channel epifluorescence microscopy (red, green, blue). Cell density per mm³ and their viability as a function of the distance from the scaffold surface were calculated. This analysis was repeated 11 days after seeding.

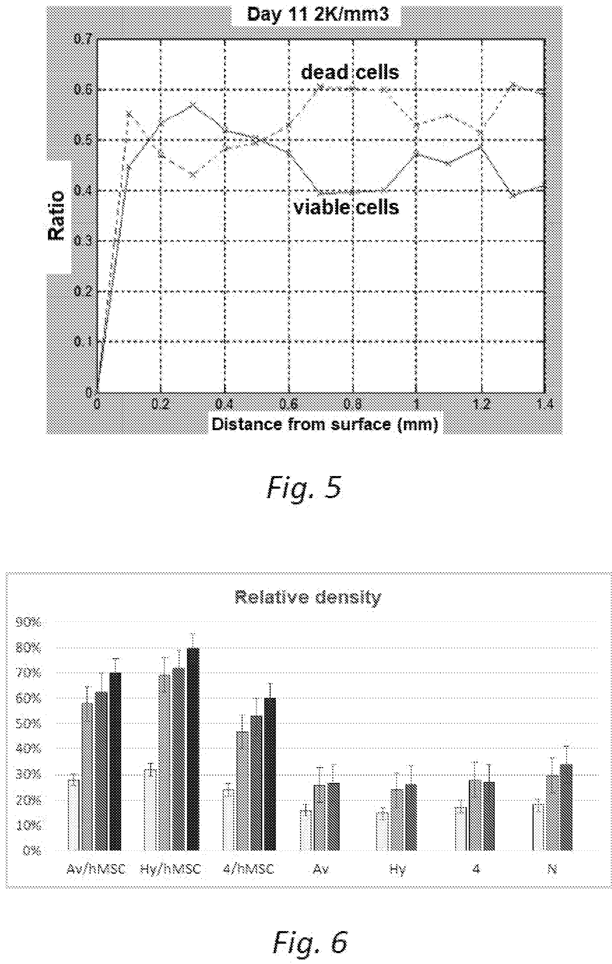

The analysis of cell distribution in the scaffolds showed homogeneous colonization of the material with adherent and well distributed cells in all longitudinal and transverse sections of the scaffold. Furthermore, total cell density and viable cell density tend to increase at the center of the scaffold with respect to the surface, while the percentage of viable cells is constant with respect to the distance from the scaffold surface: Fig. 5 shows the percentage of viable cells (solid line) and dead cells (dashed line) with respect to the minimum distance from the scaffold surface at 11 days from seeding.

From the viability analysis of human mesenchymal cells, it was observed that:
- the scaffold made with a specimen of Sample 4 shows excellent cell viability parameters;
- the percentage and density of viable cells increase significantly over the course of a week.

From the analysis of the *in vitro* test, it is concluded that the scaffold tested is biocompatible and forms a more than adequate support to accommodate the seeding, proliferation, and development of human mesenchymal cells.

The test was repeated under identical conditions but using human mesenchymal stem cells (hMSC) at a concentration of 3,000 cells/mm³, and it was observed that with increasing amounts of hMSCs seeded, there was an increase in the percentage and density of viable cells.

### EXAMPLE 8

This example relates to and implant surgery of a scaffold of the invention at mandibular level in an animal model - *In vivo* test.

*In vivo* tests were performed on New Zealand white rabbits weighing more than 3 kg and in healthy conditions. The animals underwent the following procedure under general anaesthesia with isoflurane: 1) transcervical exposure of the lower edge of the mandible; 2) creation of a bilateral lower marginal defect of 3 × 3 × 5 mm, and implantation at this level of scaffolds obtained from Sample 4; 3) muscle cuff suture around the implantation site, after removal of the periosteum; 4) awakening of the animals and postoperative monitoring.

The animals were subjected to antibiotic prophylaxis, analgesic therapy, specialist veterinary clinical monitoring, and biochemical monitoring (blood count with leukocyte formula) every two weeks. An *in vivo* radiological study was also performed by means of cone-beam computed tomography (CT) scan, with and without contrast medium, with biweekly periodicity. The radiological images obtained were imported into a 3D-modeling software (Mimics^{®}/3-matic^{®} Materialise^{®}) and the signal density at the implant site level was measured at each CT measurement; the density was expressed with respect to the value measured by preoperative CT.

Figure 6 shows the data of reconstruction trend over time obtained with three scaffolds tested, with and without human mesenchymal cell (hMSC) insemination, compared to an unreconstructed site. The groups of bars in the histograms represent respectively: the data for Sample Hy with insemination (Hy/hMSC), for Sample 4 with insemination (4/hMSC) and the average of the results obtainable with the two reconstructions with insemination (Av/hMSC); the data for Sample Hy without insemination (Hy), for Sample 4 without insemination (4) and the average of the results obtainable with the two reconstructions without insemination (Av); and the data for an unreconstructed site (N). In each group of bars, the data are reported for increasing times going from left to right: in particular, the data refer to the measurements at 30, 60 and 90 days for the samples Av, Hy, 4 and N, and at 30, 60, 90 and 120 days for Av/hMSC, Hy/hMSC and 4/hMSC, respectively.

Serial radiological analysis using cone-beam CT has demonstrated that the signal density at the implant site level increases progressively over time. The signal increase rate and the density value obtained are greater in case of defects repaired with scaffolds and hMSCs with respect to those repaired only with scaffolds or not repaired.

The absorption of contrast medium was also measured by comparing the previous acquisition with the one subsequent to administration of the same.

Clinical and biochemical monitoring of animals demonstrated the following:
- no subjects died during the surgical procedure and in the postoperative period;
- all subjects started to feed normally and to produce a normal urinary and fecal output by the second postoperative day;
- none of the subjects had an infection or clinically demonstrable abnormal inflammation of the surgical site;
- hemoglobinemia, hematocrit, leukocyte and leukocyte subpopulations counts, and platelet counts values did not undergo any clinically relevant changes during the follow-up.

### COMMENT ON THE RESULTS

The results of the tests confirm that the integrated core-shell bioactive structures of the invention exhibit an excellent set of properties in view of application as a scaffold for bone and osteochondral regrowth.

Firstly, all the reagents used are biocompatible and widely used for biomedical applications, and the production of these porous bodies does not require the use of additives or catalysts, thus avoiding possible risks of non-biocompatibility or toxicity; besides, the reagents used in the production of the shell portion of these structures are water-soluble so that no other solvent is required.

Mechanical tests demonstrate that the scaffold material made with these structures has optimal properties for a temporary replacement of a regrowing bone tissue.

Finally, the cellular regrowth and osteogenic differentiation tests, both *in vitro* and *in vivo,* confirm the properties of the scaffolds of the invention in favouring the osseointegration of prosthetic implants by reducing the healing time of bone or osteochondral tissues.

## Claims

1. Integrated core-shell bioactive structure for the regeneration of bone and osteochondral tissues, consisting of:
- a porous core made of a biocompatible and biodegradable thermoplastic polymer selected from polylactic acid (PLA), polyglycolic acid (PGA), polycaprolactone (PCL) and mixtures thereof, in an amount between 1 and 99% by weight of the structure; and
- a core coating, in an amount between 1 and 99% by weight of the structure, comprising a hydrogel formed by reaction of a polypeptide either derived from natural tissues or synthetic, a water-soluble polymer functionalized so as to be capable of reacting with the polypeptide, and an optionally aminated polysaccharide, with a weight average molecular weight greater than 3 kDa.

2. Integrated bioactive structure according to claim 1, wherein:
- said polypeptide is present in the core coating material in an amount between 48.6% and 80.2% by weight, calculated on the sum of the polypeptide, water-soluble polymer and polysaccharide components, and is a gelatin obtained by hydrolysis of collagen extracted from skin, bones and connective tissues of animal waste;
- said functionalized water-soluble polymer is present in the core coating material in an amount between 11.4% and 18.8% by weight, calculated on the sum of the polypeptide, water-soluble polymer and polysaccharide components, and is selected from polyethylene glycol (PEG), polyvinylpyrrolidone (PVP) and polyvinyl alcohol (PVA);
- said polysaccharide is present in the core coating material in an amount between 1% and 40% by weight, calculated on the sum of the polypeptide, water-soluble polymer and polysaccharide components, and is selected from dextran, aminated dextran, chitosan, aminated chitosan, and mixtures thereof.

3. Integrated bioactive structure according to claim 2, wherein said water-soluble polymer is polyethylene glycol diglycidyl ether, compound of formula: 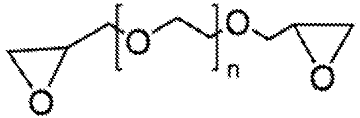 wherein n is an integer lower than 30, and preferably between 5 and 9.

4. Integrated bioactive structure according to any one of the preceding claims, wherein the core coating further contains one or more components selected from:
- mesenchymal stromal cells deriving from bone marrow, adipose tissue or umbilical cord in an amount between 1000 and 10000 cell/mm³ of the porous body;
- a growth factor;
- antibiotics, drugs or medicines useful in the treatment of bone lesions and/or in the regeneration of tissues;
- additives for modifying the stiffness or degradability of the structure, selected from complexes of calcium, calcium phosphate, calcium carbonate, decellularized and pulverized bone material and nanometric hydroxyapatite powders.

5. Integrated bioactive structure according to claim 4, wherein said growth factor is selected from human platelet lysate (HPL), bone morphogenetic protein (BMP), growth factor of the vascular endothelium (VEGF) and mixtures thereof.

6. Process for producing a structure of any one of claims 1 to 5, comprising the following steps:
a) providing a porous body made of polylactic acid, polyglycolic acid, polycaprolactone or a mixture thereof, which has open and interconnected porosities throughout the body itself, and a porosity between 30 and 95%;
b) preparing an aqueous solution comprising a polypeptide either derived from natural tissues or synthetic, a water-soluble polymer functionalized so as to be capable of reacting with the polypeptide, and an optionally aminated polysaccharide, with a weight average molecular weight greater than 3 kDa, and allowing the system to react under stirring at a temperature between 20 and 70 °C for a time between 10 minutes and 1 hour;
c) immersing the core prepared in step a) in the solution prepared in step b) and then degassing the solution by subjecting it to at least one evacuation cycle at a pressure P ≤ 0.1 mbar and exposure to an inert gas atmosphere, then maintaining the solution in said inert atmosphere at a temperature between 20 and 70 °C for a time between 30 minutes and 2 hours;
d) extracting the core from the still fluid solution and allowing the layer of solution adherent to the core surface to gel, or waiting for the solution to gel and extracting a hydrogel incorporating the core from the container of the initial solution;
e) freezing the body consisting of the core coated by wet hydrogel at a temperature below -4 °C and freeze-drying it obtaining the hydrogel drying;
f) completing the hydrogel cross-linking reaction in an oven under vacuum at a temperature between 30 and 70 °C for a time between 2 and 6 hours.

7. Process according to claim 6, wherein step a) is carried out by dispersing in a first melted material, selected from polylactic acid, polyglycolic acid, polycaprolactone or a mixture thereof, particles of a second material having a melting temperature higher than that of the first material, wherein said second material is selected from a water-insoluble superabsorbent cross-linked polymer, a water-soluble material and mixtures thereof, and wherein the particles of the second material have a particle of size between 5 and 200 µm and are used in a weight ratio between the second and the first material of between 10:90 and 50:50.

8. Process according to claim 7, wherein said superabsorbent cross-linked polymer is sodium polyacrylate.

9. Process according to claim 7, wherein said water-soluble material is an inorganic salt.

10. Process according to claim 6, wherein step a) is carried out with 3D printing techniques.

11. Process according to any one of claims 6 to 10 wherein step b) is carried out with the following series of operations:
b.1) dissolving the polypeptide in distilled water, at a temperature between 20 and 70 °C;
b.2) adding the functionalized water-soluble polymer to the solution obtained in step a) and allowing the system to react at a temperature between 20 and 70 °C for a time between 5 and 30 minutes;
b.3) adding the long chain polysaccharide, selected from optionally aminated dextran and chitosan, or a mixture thereof, to the solution obtained in step b.2), and allowing the system to react at a temperature between 20 and 70 °C for a time between 5 minutes and 1 hour.

12. Process according to any one of claims 6 to 11, wherein the core is extracted from the fluid solution and allowed to rest for a time between 30 minutes and 6 hours at a temperature below 30 °C obtaining a layer of wet hydrogel which coats the external and internal walls of the core.

## Patentansprüche

1. Integrierte bioaktive Kern-Schale-Struktur zur Regeneration von Knochen- und osteochondralen Geweben, bestehend aus:
- einem porösen Kern aus einem biokompatiblen und biologisch abbaubaren thermoplastischen Polymer, ausgewählt aus Polymilchsäure (PLA), Polyglykolsäure (PGA), Polycaprolacton (PCL) und Mischungen davon, in einer Menge zwischen 1 und 99 Gew.-% der Struktur; und
- einer Kernbeschichtung in einer Menge zwischen 1 und 99 Gew.-% der Struktur, umfassend ein Hydrogel, das durch Reaktion eines Polypeptids, das entweder aus natürlichen oder synthetischen Geweben stammt, einem wasserlöslichen Polymer, das so funktionalisiert ist, dass es mit dem Polypeptid reagieren kann und einem gegebenenfalls aminierten Polysaccharid mit einem gewichtsmittleren Molekulargewicht von mehr als 3 kDa gebildet wird.

2. Integrierte bioaktive Struktur nach Anspruch 1, wobei:
- das Polypeptid in dem Kernbeschichtungmaterial in einer Menge zwischen 48,6 und 80,2 Gew.-% vorhanden ist, berechnet auf die Summe der Polypeptid-, wasserlöslichen Polymer- und Polysaccharidkomponenten, und eine Gelatine ist, die durch Hydrolyse von Kollagen aus Haut-, Knochen- und Bindegewebe von tierischen Abfällen gewonnen wird;
- das funktionalisierte wasserlösliche Polymer im Kernbeschichtungsmaterial in einer Menge zwischen 11,4 und 18,8 Gew.-% vorhanden ist, berechnet auf die Summe der Polypeptid-, wasserlöslichen Polymer- und Polysaccharidkomponenten, und ausgewählt ist aus Polyethylenglykol (PEG), Polyvinylpyrrolidon (PVP) und Polyvinylalkohol (PVA);
- das Polysaccharid im Kernbeschichtungsmaterial in einer Menge zwischen 1 und 40 Gew.-% vorhanden ist, berechnet auf die Summe der Polypeptid-, wasserlöslichen Polymer- und Polysaccharidkomponenten, und ausgewählt ist aus Dextran, aminiertem Dextran, Chitosan, aminiertem Chitosan und Mischungen davon.

3. Integrierte bioaktive Struktur nach Anspruch 2, wobei das wasserlösliche Polymer Polyethylenglykoldiglycidylether, eine Verbindung der folgenden Formel ist: 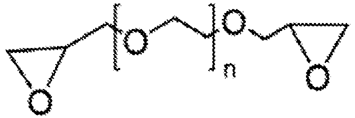 wobei n eine ganze Zahl kleiner als 30 und vorzugsweise zwischen 5 und 9 ist.

4. Integrierte bioaktive Struktur nach einem der vorstehenden Ansprüche, wobei die Kernbeschichtung ferner eine oder mehrere Komponenten enthält, ausgewählt aus:
- mesenchymalen Stromazellen aus dem Knochenmark, dem Fettgewebe oder der Nabelschnur in einer Menge zwischen 1000 und 10000 Zellen/mm³ des porösen Körpers,
- einem Wachstumsfaktor;
- Antibiotika, Drogen oder Arzneimittel, die bei der Behandlung von Knochenläsionen und/oder bei der Regeneration von Geweben nützlich sind;
- Zusatzstoffen zur Veränderung der Steifigkeit oder Zerfalls der Struktur, ausgewählt aus Calciumkomplexen, Calciumphosphat, Calciumkarbonat, dezellularisiertem und pulverisiertem Knochenmaterial und nanometrischen Hydroxyapatitpulvern.

5. Integrierte bioaktive Struktur nach Anspruch 4, wobei der Wachstumsfaktor ausgewählt ist aus humanem Blutplättchenlysat (HPL), morphogenetischem Knochenprotein (BMP), Wachstumsfaktor des vaskulären Endothels (VEGF) und Mischungen davon.

6. Verfahren zur Herstellung einer Struktur nach einem der Ansprüche 1 bis 5, umfassend die folgenden Schritte:
a) Bereitstellen eines porösen Körpers aus Polymilchsäure, Polyglykolsäure, Polycaprolacton oder einer Mischung davon, der im gesamten Körper offene und miteinander verbundene Porositäten und eine Porosität zwischen 30 und 95 % aufweist;
b) Herstellen einer wässrigen Lösung, umfassend ein Polypeptid, das entweder aus natürlichen oder synthetischen Geweben stammt, ein wasserlösliches Polymer, das so funktionalisiert ist, dass es mit dem Polypeptid reagieren kann, und ein gegebenenfalls aminiertes Polysaccharid mit einem gewichtsmittleren Molekulargewicht von mehr als 3 kDa, und Reagierenlassen des Systems unter Rühren bei einer Temperatur zwischen 20 und 70 °C für eine Zeit zwischen 10 Minuten und 1 Stunde;
c) Eintauchen des in Schritt a) hergestellten Kerns in die in Schritt b) hergestellte Lösung und anschließendes Entgasen der Lösung, indem sie mindestens einem Evakuierungszyklus bei einem Druck P ≤0,1 mbar unterzogen und einer Inertgasatmosphäre ausgesetzt wird, und anschließendes Halten der Lösung in der genannten Inertatmosphäre bei einer Temperatur zwischen 20 und 70 °C für eine Zeit zwischen 30 Minuten und 2 Stunden;
d) Extrahieren des Kerns aus der noch flüssigen Lösung und Gelierenlassen der an der Kernoberfläche haftenden Lösungsschicht oder Abwarten des Gelierens der Lösung und Extrahieren eines den Kern enthaltenden Hydrogels aus dem Behälter mit der ursprünglichen Lösung;
e) Gefrieren des Körpers, der aus dem mit nassem Hydrogel beschichteten Kern besteht, bei einer Temperatur unter -4 °C und Gefriertrocknen des Körpers unter Erhalt der Trocknung des Hydrogels;
f) Vervollständigen der Hydrogel-Vernetzungsreaktion in einem Ofen unter Vakuum bei einer Temperatur zwischen 30 und 70 °C für eine Dauer zwischen 2 und 6 Stunden.

7. Verfahren nach Anspruch 6, wobei Schritt a) durch Dispergieren von Teilchen eines zweiten Materials mit einer höheren Schmelztemperatur als die des ersten Materials in einem ersten geschmolzenen Material, ausgewählt aus Polymilchsäure, Polyglykolsäure, Polycaprolacton oder einer Mischung davon, durchgeführt wird, wobei das zweite Material ausgewählt ist aus einem wasserunlöslichen, superabsorbierenden, vernetzten Polymer, einem wasserlöslichen Material und Mischungen davon und wobei die Teilchen des zweiten Materials eine Teilchengröße zwischen 5 und 200 µm aufweisen und in einem Gewichtsverhältnis zwischen dem zweiten und dem ersten Material zwischen 10:90 und 50:50 verwendet werden.

8. Verfahren nach Anspruch 7, wobei das superabsorbierende vernetzte Polymer Natriumpolyacrylat ist.

9. Verfahren nach Anspruch 7, wobei das wasserlösliche Material ein anorganisches Salz ist.

10. Verfahren nach Anspruch 6, wobei der Schritt a) mit 3D-Drucktechniken durchgeführt wird.

11. Verfahren nach einem der Ansprüche 6 bis 10, wobei Schritt b) mit der folgenden Reihe von Arbeitsgängen durchgeführt wird:
b.1) Auflösen des Polypeptids in destilliertem Wasser bei einer Temperatur zwischen 20 und 70 °C;
b.2) Zugeben des funktionalisierten wasserlöslichen Polymers zu der in Schritt a) erhaltenen Lösung und Reagierenlassen des Systems bei einer Temperatur zwischen 20 und 70 °C für eine Dauer zwischen 5 und 30 Minuten;
b.3) Zugeben des langkettigen Polysaccharids, ausgewählt aus gegebenenfalls aminiertem Dextran und Chitosan oder einer Mischung davon, zu der in Schritt b.2) erhaltenen Lösung und Reagierenlassen des Systems bei einer Temperatur zwischen 20 und 70 °C für eine Zeit zwischen 5 Minuten und 1 Stunde.

12. Verfahren nach einem der Ansprüche 6 bis 11, wobei der Kern aus der flüssigen Lösung extrahiert und für eine Zeit zwischen 30 Minuten und 6 Stunden bei einer Temperatur unter 30 °C ruhen gelassen wird, wodurch eine Schicht aus nassem Hydrogel erhalten wird, die die Außen- und Innenwände des Kerns beschichtet.

## Revendications

1. Structure bioactive intégrée de type virole de coeur pour la régénération des tissus osseux et ostéochondraux, composée de :
- un coeur poreux constitué d'un polymère thermoplastique biocompatible et biodégradable choisi parmi l'acide polylactique (PLA), l'acide polyglycolique (PGA), le polycaprolactone (PCL) et leurs mélanges, dans une proportion comprise entre 1 et 99 % en poids de la structure ; et
- un revêtement central, en quantité comprise entre 1 et 99 % en poids de la structure, comprenant un hydrogel formé par la réaction d'un polypeptide dérivé de tissus naturels ou synthétiques, d'un polymère soluble dans l'eau fonctionnalisé de manière à pouvoir réagir avec le polypeptide, et d'un polysaccharide facultativement aminé, d'un poids moléculaire moyen en poids supérieur à 3 kDa.

2. Structure bioactive intégrée selon la revendication 1, dans laquelle :
- ledit polypeptide est présent dans le matériau de revêtement du coeur en une quantité comprise entre 48,6 % et 80,2 % en poids, calculée sur la somme des composants polypeptides, polymères hydrosolubles et polysaccharides, et est une gélatine obtenue par hydrolyse du collagène extrait de la peau, des os et des tissus conjonctifs de déchets d'origine animale ;
- ledit polymère hydrosoluble fonctionnalisé est présent dans le matériau de revêtement du coeur en une quantité comprise entre 11,4 % et 18,8 % en poids, calculée sur la somme des composants polypeptides, polymères hydrosolubles et polysaccharides, et est choisi parmi le polyéthylène glycol (PEG), le polyvinylpyrrolidone (PVP) et l'alcool polyvinylique (PVA) ;
- ledit polysaccharide est présent dans le matériau de revêtement du coeur en une quantité comprise entre 1 % et 40 % en poids, calculée sur la somme des composants polypeptidiques, polymères hydrosolubles et polysaccharides, et est choisi parmi le dextran, le dextran aminé, le chitosane, le chitosane aminé, et leurs mélanges.

3. Structure bioactive intégrée selon la revendication 2, dans laquelle ledit polymère hydrosoluble est un éther diglycidylique de polyéthylène glycol, composé de formule : 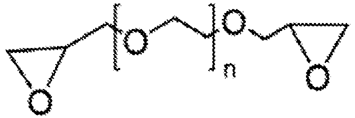 où n est un nombre entier inférieur à 30, et de préférence compris entre 5 et 9.

4. Structure bioactive intégrée selon l'une quelconque des revendications précédentes, dans laquelle le revêtement du coeur contient en outre un ou plusieurs composants choisis parmi :
- des cellules stromales mésenchymateuses provenant de la moelle osseuse, du tissu adipeux ou du cordon ombilical dans une quantité comprise entre 1 000 et 10 000 cellules/mm³ du corps poreux ;
- un facteur de croissance ;
- des antibiotiques, drogues ou médicaments utiles au traitement des lésions osseuses et/ou à la régénération des tissus ;
- des additifs destinés à modifier la rigidité ou la dégradabilité de la structure, choisis parmi les complexes de calcium, phosphate de calcium, carbonate de calcium, matière osseuse décellularisée et pulvérisée et poudres d'hydroxyapatite nanométrique.

5. Structure bioactive intégrée selon la revendication 4, dans laquelle ledit facteur de croissance est choisi parmi le lysat plaquettaire humain (HPL), la protéine morphogénétique osseuse (BMP), le facteur de croissance de l'endothélium vasculaire (VEGF) et leurs mélanges.

6. Procédé de fabrication d'une structure selon l'une des revendications 1 à 5, comprenant les étapes suivantes :
a) fournir un corps poreux en acide polylactique, en acide polyglycolique, en polycaprolactone ou un mélange de ceux-ci, qui présente des porosités ouvertes et interconnectées dans l'ensemble du corps lui-même, et une porosité comprise entre 30 et 95 % ;
b) préparer une solution aqueuse comprenant un polypeptide dérivé de tissus naturels ou synthétiques, un polymère hydrosoluble fonctionnalisé de manière à pouvoir réagir avec le polypeptide, et un polysaccharide éventuellement aminé, d'un poids moléculaire moyen en poids supérieur à 3 kDa, et laisser le système réagir sous agitation à une température comprise entre 20 et 70 °C pendant une durée comprise entre 10 minutes et 1 heure ;
c) immerger le coeur préparé à l'étape a) dans la solution préparée à l'étape b), puis dégazer la solution en la soumettant à au moins un cycle d'évacuation à une pression P ≤0,1 mbar et à l'exposition à une atmosphère de gaz inerte, puis maintenir la solution dans ladite atmosphère inerte à une température comprise entre 20 et 70 °C pendant une durée comprise entre 30 minutes et 2 heures ;
d) extraire le coeur de la solution encore fluide et permettre à la couche de solution adhérant à la surface du coeur de se gélifier, ou attendre que la solution se gélifie et extraire un hydrogel incorporant le coeur du récipient de la solution initiale ;
e) congeler le corps constitué du coeur enrobé d'hydrogel humide à une température inférieure à -4 °C et le lyophiliser en obtenant le séchage de l'hydrogel ;
f) achever la réaction de réticulation de l'hydrogel dans une étuve sous vide à une température comprise entre 30 et 70 °C pendant une durée comprise entre 2 et 6 heures.

7. Procédé selon la revendication 6, dans lequel l'étape a) est réalisée en dispersant dans un premier matériau fondu, choisi parmi l'acide polylactique, l'acide polyglycolique, le polycaprolactone ou un mélange de ceux-ci, des particules d'un second matériau ayant une température de fusion supérieure à celle du premier matériau, dans lequel ce second matériau est choisi parmi un polymère réticulé superabsorbant insoluble dans l'eau, un matériau soluble dans l'eau et leurs mélanges, et dans lequel les particules du second matériau ont une taille comprise entre 5 et 200 µm et sont utilisées dans un rapport de poids entre le second et le premier matériau compris entre 10/90 et 50/50.

8. Procédé selon la revendication 7, dans lequel ledit polymère réticulé superabsorbant est un polyacrylate de sodium.

9. Procédé selon la revendication 7, dans lequel ladite matière soluble dans l'eau est un sel inorganique.

10. Procédé selon la revendication 6, dans lequel l'étape a) est réalisée à l'aide de techniques d'impression 3D.

11. Procédé selon l'une quelconque des revendications 6 à 10, dans lequel l'étape b) est réalisée avec la série d'opérations suivante :
b.1) dissoudre le polypeptide dans de l'eau distillée, à une température comprise entre 20 et 70 °C ;
b.2) ajouter le polymère hydrosoluble fonctionnalisé à la solution obtenue à l'étape a) et laisser le système réagir à une température comprise entre 20 et 70 °C pendant une durée comprise entre 5 et 30 minutes ;
b.3) ajouter le polysaccharide à longue chaîne, choisi parmi le dextrane et le chitosane éventuellement aminés, ou un mélange de ceux-ci, à la solution obtenue à l'étape b.2), et laisser le système réagir à une température comprise entre 20 et 70 °C pendant une durée comprise entre 5 minutes et 1 heure.

12. Procédé selon l'une des revendications 6 à 11, dans lequel le coeur est extrait de la solution fluide et laissé au repos pendant une durée comprise entre 30 minutes et 6 heures à une température inférieure à 30 °C, ce qui permet d'obtenir une couche d'hydrogel humide qui recouvre les parois externes et internes du coeur.
